(19) Europäisches Patentamt
European Patent Office
Office européen des brevets

(11) **EP 1 965 913 B1**

(12) **FASCICULE DE BREVET EUROPEEN**

(45) Date de publication et mention
de la délivrance du brevet:
**15.04.2009 Bulletin 2009/16**

(51) Int Cl.:
***B01J 29/80*** *(2006.01)*        ***B01J 29/068*** *(2006.01)*
***C07C 5/27*** *(2006.01)*

(21) Numéro de dépôt: **06830979.8**

(22) Date de dépôt: **03.11.2006**

(86) Numéro de dépôt international:
**PCT/FR2006/002361**

(87) Numéro de publication internationale:
**WO 2007/080238 (19.07.2007 Gazette 2007/29)**

(54) **CATALYSEUR BIZEOLITHIQUE COMPRENANT UN METAL DU GROUPE VIII ET UN METAL DU GROUPE IIIA ET SON UTILISATION EN ISOMERISATION DES COMPOSES C8 AROMATIQUES**

ZEOLITH-DOPPELKATALYSATOR ENTHALTEND EIN METALL DER GRUPPE VIII UND EIN METALL DER GRUPPE IVA, UND SEINE VERWENDUNG BEI DER ISOMERISIERUNG VON AROMATISCHEN C8-VERBINDUNGEN

DUAL ZEOLITE CATALYST COMPRISING A GROUP VIII METAL AND A GROUP IVA METAL AND ITS USE IN ISOMERIZATION OF AROMATIC C8 COMPOUNDS

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **22.12.2005 FR 0513175**

(43) Date de publication de la demande:
**10.09.2008 Bulletin 2008/37**

(73) Titulaire: **Institut Français du Pétrole**
**92852 Rueil Malmaison Cedex (FR)**

(72) Inventeurs:
• **GUILLON, Emmanuelle**
**F-69390 Vernaison (FR)**
• **SANCHEZ, Eric**
**F-69230 Saint Genis Laval (FR)**
• **LACOMBE, Sylvie**
**F-69230 Saint Genis Laval (FR)**

(56) Documents cités:
**WO-A-99/28031           US-A- 4 467 129**
**US-A- 4 537 754          US-A- 6 057 486**
**US-B1- 6 872 866**

## Description

Domaine technique

La présente invention se rapporte à un .catalyseur formé d'au moins deux zéolithes distinctes, dont l'une est une zéolithe de type structural EUO, et utilisable par exemple dans les réactions de transformation des hydrocarbures aromatiques. De manière plus précise, elle concerne un catalyseur d'isomérisation des composés aromatiques comportant huit atomes de carbone par molécule. La présente invention concerne aussi l'utilisation dudit catalyseur dans un procédé d'isomérisation d'une charge comprenant des composés aromatiques à 8 atomes de carbone par molécule:

Art antérieur

Selon les procédés connus d'isomérisation des composés aromatiques à huit atomes de carbone (AC8), une charge généralement pauvre en paraxylène par rapport à l'équilibre thermodynamique du mélange (c'est-à-dire dont la teneur en paraxylène est nettement inférieure à celle du mélange à l'équilibre thermodynamique à la température considérée, ce mélange comprenant au moins un composé choisi dans le groupe formé par le métaxylène, l'orthoxylène, le paraxylène et l'éthylbenzène) et généralement riche en éthylbenzène par rapport à ce même mélange à l'équilibre thermodynamique est introduite dans un réacteur contenant au moins un catalyseur, dans des conditions de température et de pression adéquates pour obtenir une composition, à la sortie dudit réacteur, en composés aromatiques à huit atomes de carbone la plus proche possible de la composition dudit mélange à l'équilibre thermodynamique à la température du réacteur. De ce mélange, on sépare ensuite le paraxylène et éventuellement le métaxylène ou l'orthoxylène qui sont les isomères recherchés car ils présentent un intérêt important notamment pour l'industrie des fibres synthétiques.

Les catalyseurs utilisés pour la mise en oeuvre d'un procédé d'isomérisation de composés aromatiques à huit atomes de carbone sont généralement des catalyseurs zéolithiques. Les catalyseurs de l'état de la technique, en particulier les catalyseurs à base de zéolithe mordénite, ne donnent que des performances catalytiques médiocres car ils conduisent à des réactions parasites non négligeables générant des pertes. Par exemple, on peut citer, parmi ces réactions secondaires, l'ouverture de cycles naphténiques suivie ou non de craquage (pertes vers les paraffines) ou encore les réactions de dismutation et de transalkylation des aromatiques à huit atomes de carbone (pertes vers des composés aromatiques non recherchés) ou bien encore l'hydrogénation des composés aromatiques (pertes vers les naphtènes). Des catalyseurs à base de zéolithe ZSM-5, seule ou en mélange avec d'autres zéolithes comme la mordénite par exemple, ont déjà été utilisés mais ne donnent pas, non plus, des performances catalytiques optimales. Plus récemment, il a été proposé un catalyseur à base d'une zéolithe de type structural EUO (EP-A1-0.923.987).

Aussi, la présente invention se propose de fournir un nouveau catalyseur présentant une composition telle que lorsqu'il est utilisé pour l'isomérisation des composés aromatiques à 8 atomes de carbone par molécule, les réactions secondaires sont limitées, réduisant en conséquence les pertes.

Résumé

La présente invention a pour objet un catalyseur comportant au moins une zéolithe de type structural EUO, au moins une zéolithe choisie parmi la zéolithe IM-5 et les zéolithes de type structural MFI, MOR, BEA et MTW, au moins un métal du groupe VIII de la classification périodique des éléments, au moins un métal du groupe IIIA de la classification périodique des éléments et au moins une matrice minérale poreuse. Chacune des zéolithes comprises dans le catalyseur selon l'invention contient du silicium et au moins un élément T choisi dans le groupe formé par l'aluminium, le fer, le gallium et le bore, de préférence l'aluminium.

La présente invention concerne également l'utilisation dudit catalyseur dans un procédé d'isomérisation d'une charge comprenant des composés aromatiques à huit atomes de carbone par molécule.

Intérêt

Il a été découvert, de manière surprenante, qu'un catalyseur composite comprenant l'association d'au moins une zéolithe de type structural EUO, d'au moins une zéolithe choisie parmi la zéolithe IM-5 et les zéolithes de type structural MFI, MOR, BEA et MTW, au moins un métal du groupe VIII et au moins un métal du groupe IIIA conduit à des performances catalytiques améliorées dans les réactions d'isomérisation des composés aromatiques à 8 atomes de carbone par molécule. En particulier, le catalyseur selon l'invention limite notablement les réactions secondaires, générant ainsi de moindres pertes, par rapport aux catalyseurs de l'état de la technique.

De plus, en ajustant la quantité relative des deux zéolithes, celle de type structural EUO et celle choisie parmi la zéolithe IM-5 et les zéolithes de type structural MFI, MOR, BEA et MTW, dans le catalyseur selon l'invention, il est possible de traiter une très large gamme de mélanges de charges hydrocarbonées.

Description

La présente invention a pour objet un catalyseur comportant au moins une zéolithe de type structural EUO, au moins une zéolithe choisie parmi la zéolithe IM-5 et les zéolithes de type structural MFI, MOR, BEA et MTW, au moins un métal du groupe VIII de la classification périodique des éléments, au moins un métal du groupe IIIA de la classification périodique des éléments et au moins une matrice minérale poreuse.

Conformément à l'invention, le catalyseur comporte au moins deux zéolithes de type structural différent.

Les zéolithes présentes dans le catalyseur selon l'invention comprennent du silicium et au moins un élément T choisi dans le groupe formé par l'aluminium, le fer, le gallium et le bore, de préférence l'aluminium. Elles sont, de préférence, pratiquement totalement, sous forme acide.

La zéolithe de type structural EUO, présente dans le catalyseur selon l'invention, est déjà décrite dans l'état de la technique. Elle présente un réseau microporeux monodimensionnel, dont le diamètre des pores est de 4,1 x 5,7 Å (1 Å = 1 Angström = $10^{-10}$ m) (« Atlas of Zeolite Framework Types », W.M. Meier, D.H. Olson et Ch. Baerlocher, 5ème Edition, 2001). D'autre part, N.A. Briscoe et al ont enseigné dans un article de la revue Zeolites (1988, 8, 74) que ces canaux monodimensionnels possèdent des poches latérales de profondeur 8,1 Å et de diamètre 6,8 x 5,8 Å. La zéolithe de type structural EUO regroupe les zéolithes EU-1 (EP-B1-42 226), ZSM-50 (US 4.640.829) et TPZ-3 (EP-A1-51 318). La zéolithe de type structural EUO, présente dans le catalyseur selon l'invention, est de préférence une zéolithe EU-1. Ladite zéolithe de type structural EUO est caractérisée par un rapport atomique Si/T, de préférence un rapport atomique Si/Al, d'au moins 5, avantageusement compris entre 5 et 100. Ladite zéolithe de type structural EUO est au moins en partie, de préférence pratiquement totalement, sous forme acide, c'est-à-dire sous forme hydrogène $H^+$, la teneur en sodium étant de préférence telle que le rapport atomique Na/T est inférieur à 0,1, de manière préférée inférieur à 0,05 et de manière encore plus préférée inférieur à 0,01. Un mode de synthèse d'une zéolithe EU-1 est décrit dans le brevet EP-B1-42 226. Un mode de synthèse d'une zéolithe ZSM-50 est décrit dans le brevet US 4.640.829. Un mode de synthèse d'une zéolithe TPZ-3 est décrit dans la demande de brevet EP-A1-51 318.

Le catalyseur selon l'invention comporte également au moins une zéolithe choisie parmi la zéolithe IM-5 et les zéolithes de type structural MFI, MOR, BEA et MTW. De préférence, ladite zéolithe choisie parmi la zéolithe IM-5 et les zéolithes de type structural MFI, MOR, BEA et MTW est caractérisée par un rapport atomique Si/T, de préférence un rapport atomique Si/Al, compris entre 2 et 250, de préférence compris entre 5 et 150 et de manière très préférée compris entre 10 et 80. La teneur en sodium est inférieure à 0,2 % poids, de préférence inférieure à 0,1 % poids et de manière très préférée inférieure à 0,05 % poids par rapport au poids total de zéolithe sèche. Les zéolithes de type structural MFI, MOR, BEA et MTW sont répertoriées dans l'atlas des Zéolithes ("Atlas of Zeolite Framework Types", W. M Meier, D. H. Olson et Ch. Baerlocher, 5th revised edition, 2001) et sont synthétisées selon les méthodes décrites dans les références citées dans cet ouvrage ou par toute autre méthode décrite dans la littérature ouverte à l'Homme du métier. Toute zéolithe commerciale peut par ailleurs être utilisée pour préparer le catalyseur selon l'invention. De manière très avantageuse, la zéolithe choisie parmi la zéolithe IM-5 et les zéolithes de type structural MFI, MOR, BEA et MTW est choisie parmi les zéolithes IM-5, ZSM-5, mordénite, beta et ZSM-12. La zéolithe IM-5 est décrite dans les brevets EP-B-0.946.416 et US 6.136.290.

Les cristaux de la zéolithe de type structural EUO et ceux de la zéolithe choisie parmi la zéolithe IM-5 et les zéolithes de type structural MFI, MOR, BEA et MTW sont bien distincts les uns des autres : ils ne se recouvrent pas.

Le rapport atomique Si/T, de préférence le rapport atomique Si/Al, de la zéolithe de type structural EUO et celui de la zéolithe choisie parmi la zéolithe IM-5 et les zéolithes de type structural MFI, MOR, BEA et MTW sont ceux obtenus à l'issue de la synthèse desdites zéolithes ou bien obtenus après des traitements post-synthèse d'extraction d'une partie des atomes T, dits traitements de désalumination lorsque l'élément T est l'aluminium, bien connus de l'homme du métier, tels que et à titre non exhaustif les traitements hydrothermiques suivis ou non d'attaques acides ou bien encore les attaques acides directes par des solutions d'acides minéraux ou organiques visant à extraire une partie des atomes T, de préférence une partie des atomes d'aluminium, de la charpente zéolithique.

Le rapport atomique Si/T, de préférence le rapport atomique Si/Al de la zéolithe de type structural EUO et de la zéolithe choisie parmi la zéolithe IM-5 et les zéolithes de type structural MFI, MOR, BEA et MTW entrant dans la composition du catalyseur selon l'invention ainsi que la composition chimique dudit catalyseur sont déterminés par fluorescence X et absorption atomique.

Les zéolithes entrant dans la composition du catalyseur selon l'invention peuvent être calcinées et échangées par au moins un traitement par une solution d'au moins un sel d'ammonium de manière à obtenir la forme ammonium des zéolithes qui une fois calcinée conduisent à la forme hydrogène desdites zéolithes.

Les zéolithes entrant dans la composition du catalyseur selon l'invention sont au moins en partie, de préférence pratiquement totalement, sous forme acide, c'est-à-dire sous forme hydrogène ($H^+$). Le rapport atomique Na/T est généralement inférieur à 10% et de préférence inférieur à 5% et de manière encore plus préférée inférieur à 1 %.

Le catalyseur selon l'invention comprend en outre au moins un métal du groupe VIII de la classification périodique des éléments et au moins un métal du groupe IIIA de la classification périodique des éléments. La teneur pondérale de chaque métal est de préférence comprise entre 0,01 et 5 %, de manière préférée comprise entre 0,05 et 3 % et de manière très préférée compris entre 0,2 et 2% poids par rapport au poids total dudit catalyseur. Préférentiellement, le métal du groupe VIII est un métal noble, de préférence le platine. Préférentiellement, le métal du groupe IIIA est l'indium. Le catalyseur selon l'invention comporte éventuellement en outre au moins un métal du groupe IVA, préférentiellement à une teneur comprise entre 0,01 et 5 % et de manière très préférée comprise entre 0,5 et 3% poids par rapport au poids total du catalyseur. Très avantageusement, ledit métal du groupe IVA est l'étain. Ledit métal du groupe IVA permet avantageusement d'accroître la stabilité du catalyseur selon l'invention.

La matrice minérale poreuse, présente préférentiellement à une teneur comprise entre 10 et 98 %, de préférence entre 20 et 95 %, de manière plus préférée entre 60 et 95% poids par rapport au poids total de catalyseur, est choisie généralement parmi les éléments du groupe formé par les argiles (par exemple parmi les argiles naturelles telles que le kaolin, la sepiolite, l'attapulgite ou la bentonite), la magnésie, les alumines, les silices, l'oxyde de titane, l'oxyde de bore, la zircone, les phosphates d'aluminium, les phosphates de titane, les phosphates de zirconium, les silice-alumines amorphes et le charbon, de préférence parmi les éléments du groupe formé par les alumines, les argiles, les mélanges d'alumine et de silice et les mélanges d'alumine et de silice-alumine, de manière encore plus préférée parmi les alumines et notamment l'alumine gamma.

De manière générale et selon un premier mode de réalisation de la préparation du catalyseur selon l'invention, le catalyseur est préparé par mélange d'au moins une zéolithe de type structural EUO et d'au moins une zéolithe choisie parmi la zéolithe IM-5 et les zéolithes de type structural MFI, MOR, BEA et MTW, lesdites zéolithes se trouvant à l'état de poudre. Le mélange desdites zéolithes est réalisé par toutes les techniques de mélange de poudres connues de l'Homme du métier. Une fois le mélange des poudres de zéolithes, réalisé, le mélange est mis en forme par toute technique connue de l'Homme du métier. Il peut en particulier être mélangé à une matrice minérale poreuse, généralement amorphe, par exemple à une poudre humide de gel d'alumine. Le mélange est ensuite mis en forme, par exemple par extrusion au travers d'une filière. La mise en forme peut être réalisée avec d'autres matrices que l'alumine, telles que par exemple la magnésie, les silice-alumines amorphes, les argiles naturelles (kaolin, bentonite, sepiolite, attapulgite), la silice, l'oxyde de titane, l'oxyde de bore, la zircone, les phosphates d'aluminium, les phosphates de titane, les phosphates de zirconium, le charbon et leurs mélanges. On préfère utiliser des matrices contenant de l'alumine, sous toutes ses formes connues de l'Homme du métier, et de manière encore plus préférée l'alumine gamma. On peut aussi avantageusement utiliser des mélanges d'alumine et de silice, des mélanges d'alumine et de silice-alumine. D'autres techniques que l'extrusion, telles que le pastillage ou la dragéification, peuvent être utilisées. Après l'étape de mise en forme, le support catalytique obtenu est soumis à une étape de séchage réalisé à une température comprise entre 80 et 150°C puis à une étape de calcination réalisé à une température comprise entre 300 et 600°C, de préférence entre 400 et 550°C.

Les métaux appartenant aux groupes IIIA et VIII et éventuellement le métal du groupe IVA sont déposés, sur au moins un support catalytiques, après la mise en forme des zéolithes exemptes de métaux, par tout procédé connu de l'homme du métier et permettant le dépôt du métal sur le support catalytique. On désigne par "support catalytique", le mélange d'au moins une zéolithe de type structural EUO, d'au moins une zéolithe choisie parmi la zéolithe IM-5 et les zéolithes de type structural MFI, MOR, BEA et MTW (zéolithes exemptes de métaux) avec au moins une matrice minérale poreuse après mise en forme, séchage et calcination, comme décrit ci-dessus. Ledit support catalytique du catalyseur de la présente invention renferme généralement les teneurs suivantes en matrice et zéolithes :

- 2 à 90% poids, de préférence 5 à 80% poids, de manière plus préférée de 5 à 40% poids de zéolithes telles que au moins une zéolithe soit une zéolithe de type structural EUO et au moins une zéolithe soit une zéolithe choisie parmi la zéolithe IM-5 et les zéolithes de type structural MFI, MOR, BEA et MTW,
- 10 à 98% poids, de préférence de 20 à 95% poids, de manière plus préférée de 60 à 95% poids d'au moins une matrice minérale poreuse amorphe ou mal cristallisée de type oxyde.

Selon un deuxième mode de réalisation de préparation du catalyseur de l'invention, préalablement à leur mise en forme, au moins une des zéolithes précédemment décrites, c'est-à-dire au moins une zéolithe de type structural EUO ou au moins une zéolithe choisie parmi la zéolithe IM-5 et les zéolithes de type structural MFI, MOR, BEA et MTW, comprise dans ledit catalyseur est soumise au dépôt d'au moins un métal choisi parmi les métaux des groupes IIIA et VIII, éventuellement du groupe IVA. En particulier, on peut envisager de déposer au moins un métal du groupe VIII et au moins un métal du groupe IIIA sur la même zéolithe ou déposer au moins un métal du groupe VIII sur une zéolithe et au moins un métal du groupe IIIA sur l'autre zéolithe. Les zéolithes, dont l'une au moins est chargée en métal(ux), sont mélangées. Le mélange de ces zéolithes, dont l'une au moins est chargée en métal(ux), qui sont alors à l'état de poudre est réalisé par toutes les techniques de mélange de poudres connues de l'Homme du métier.

Une fois le mélange des poudres de zéolithes, dont l'une au moins est chargée en métal(ux), réalisé, le mélange est mis en forme par toute technique connue de l'Homme du métier. Il peut en particulier être mélangé à une matrice minérale poreuse, généralement amorphe, par exemple à une poudre humide de gel d'alumine. Le mélange est ensuite mis en forme, par exemple par extrusion au travers d'une filière. La mise en forme peut être réalisée avec d'autres matrices que l'alumine, telles que par exemple la magnésie, les silice-alumines amorphes, les argiles naturelles (kaolin, bentonite; sepiolite, attapulgite), la silice, l'oxyde de titane, l'oxyde de bore, la zircone, les phosphates d'aluminium, les phosphates de titane, les phosphates de zirconium, le charbon et leurs mélanges. On préfère utiliser des matrices contenant de l'alumine, sous toutes ces formes connues de homme du métier, et de manière encore plus préférée l'alumine gamma. On peut aussi avantageusement utiliser des mélanges d'alumine et de silice, des mélanges d'alumine et de silice-alumine. D'autres techniques que l'extrusion, telles que le pastillage ou la dragéification, peuvent être utilisées.

Après l'étape de mise en forme, le produit obtenu est soumis à une étape de séchage réalisée à une température comprise entre 80 et 150°C puis à une étape de calcination réalisée à une température comprise entre 300 et 600°C, de préférence entre 400 et 550°C.

Pour le dépôt des métaux des groupes IIIA et VIII, éventuellement du groupe IVA, sur au moins une zéolithe de type structural EUO et / ou sur au moins une zéolithe choisie parmi la zéolithe IM-5 et les zéolithes de type structural MFI, MOR, BEA et MTW et/ou sur le support catalytique selon le premier ou le deuxième mode de préparation du catalyseur selon l'invention, on peut utiliser la technique d'échange cationique avec compétition où le compétiteur est de préférence le nitrate d'ammonium, le rapport de compétition entre le compétiteur et le précurseur métallique étant au moins égal à environ 5 et avantageusement compris entre 5 et 200. On peut utiliser aussi la technique d'imprégnation à sec ou de coprécipitation.

Les sources des métaux du groupe VIII qui peuvent être utilisées sont bien connues de l'Homme du métier. Par exemple, on utilisera les nitrates, les sulfates, les phosphates, les halogénures par exemple chlorures, bromures et fluorures, les carboxylates par exemple acétates et carbonates. Dans le cas du platine, on utilise préférentiellement de l'acide hexachloroplatinique. Les sources des métaux du groupe IIIA qui peuvent être utilisées sont également bien connues de homme du métier. Pour l'indium, on utilise préférentiellement le nitrate d'indium ou le chlorure d'indium. Les sources des métaux du groupe IVA qui peuvent être utilisées sont également bien connues de l'Homme du métier. Pour l'étain, on utilise préférentiellement le chlorure d'étain ou le tétrabutylétain. Le dépôt des métaux du groupe VIII et du groupe IIIA, éventuellement du groupe IVA, est suivi en général d'une calcination sous air ou oxygène, usuellement entre 300 et 600°C durant 0,5 à 10 heures, de préférence entre 350°C et 550°C durant 1 à 4 heures: On peut procéder ensuite à une réduction sous hydrogène, généralement à une température comprise entre 300 et 600°C pendant 1 à 10 heures, de préférence on opérera entre 350° et 550°C pendant 2 à 5 heures.

Selon ledit premier ou ledit deuxième mode de réalisation de préparation du catalyseur selon l'invention, on peut également- déposer les métaux non plus directement sur les zéolithes, mais sur la matrice minérale poreuse (par exemple le liant aluminique) du support catalytique, avant ou après l'étape de mise en forme, en mettant en oeuvre un échange anionique. En général après le dépôt de métal, le catalyseur est comme précédemment soumis à une calcination puis réduit sous hydrogène comme indiqué ci-dessus.

Les métaux des groupes VIII et IIIA, éventuellement du groupe IVA, sont introduits soit tous de la même façon soit par des techniques différentes, avant ou après mise en forme selon le mode de préparation du catalyseur employé et dans n'importe quel ordre. Dans le cas où la technique utilisée est celle de l'échange ionique, plusieurs échanges successifs peuvent être nécessaires pour introduire les quantités requises de métaux.

Quelle que soit le mode de préparation du catalyseur selon l'invention, on peut procéder après la calcination dudit catalyseur à une réduction sous hydrogène, généralement à une température comprise entre 300 et 600°C, de préférence entre 350 et 550°C, et pour une durée comprise entre 1 et 10 heure(s), de préférence entre 2 et 5 heures. Une telle réduction peut avoir lieu ex *situ* ou *in situ,* par rapport au lieu d'utilisation dudit catalyseur dans une réaction donnée.

La répartition entre les deux zéolithes présentes dans le catalyseur selon l'invention est telle que la teneur en zéolithe de type structural EUO peut varier de 1% à 99%, de préférence de 5 à 95% et de manière encore plus préférée de 10 à 90% en pourcentages massiques de la zéolithe de type structural EUO par rapport à l'ensemble des zéolithes introduites dans le catalyseur. De même, la teneur en zéolithe choisie parmi la zéolithe IM-5 et les zéolithes de type structural MFI, MOR, BEA et MTW varie de 1% à 99%, de préférence de 5 à 95% et de manière encore plus préférée de 10 à 90%, en pourcentages massiques de ladite zéolithe par rapport à l'ensemble des zéolithes introduites dans le catalyseur.

Le catalyseur selon la présente invention est mis en forme sous la forme de grains de différentes formes et dimensions. Il est utilisé en général sous la forme d'extrudés cylindriques ou polylobés tels que bilobés, trilobés, polylobés de forme droite ou torsadée, mais peut éventuellement être fabriqué et employé sous la forme de poudre, pastilles, de tablettes, d'anneaux, de billes, de roues.

Le catalyseur de la présente invention peut éventuellement contenir du soufre. Dans ce cas, le soufre est introduit sur le catalyseur mis en forme, calciné, contenant le ou les élément(s) cité(s) précédemment, soit *in situ* avant la réaction catalytique, soit ex *situ.* La sulfuration s'effectue en utilisant tout agent sulfurant bien connu de l'homme de métier, tel que par exemple le disulfure de diméthyle ou le sulfure d'hydrogène. La sulfuration éventuelle intervient après la réduction. Dans le cas d'une sulfuration *in situ,* la réduction, si le catalyseur n'a pas été préalablement réduit, intervient avant la sulfuration. Dans le cas d'une sulfuration *ex situ*, on effectue la réduction puis la sulfuration.

Un autre objet de l'invention est l'utilisation du catalyseur selon l'invention dans des procédés pour la conversion d'hydrocarbures. Plus précisément, la présente invention concerne un procédé d'isomérisation d'une charge comprenant des composés aromatiques à 8 atomes de carbone par molécule réalisé en présence du catalyseur selon l'invention. Ladite charge comprend un mélange de xylènes et de l'éthylbenzène. Ledit procédé est préférentiellement mis en oeuvre en phase gazeuse, en l'absence de toute phase liquide. Ledit procédé est mis en oeuvre généralement selon les conditions opératoires suivantes :

- une température comprise entre 300°C et 500°C, de préférence entre 320°C et 450°C et de manière encore plus préférée entre 340°C et 430°C,
- une pression partielle d'hydrogène comprise entre 0,3. et 1,5 MPa, de préférence entre 0,4 et 1,2 MPa et de manière encore préférée entre 0,7 et 1,2 MPa,
- une pression totale comprise entre 0,45 et 1,9 MPa, de préférence entre 0,6 et 1,5 MPa,
- une vitesse spatiale d'alimentation, exprimée en kilogramme de charge introduite par kilogramme de catalyseur et par heure, comprise entre 0,25 et 30 $h^{-1}$, de préférence entre 1 et 10 $h^{-1}$, et de manière encore plus préférée entre 2 et 6 $h^{-1}$.

Les exemples qui suivent illustrent l'invention sans pour autant en limiter la portée.

**Exemple 1 : Préparation d'un catalyseur à base d'une zéolithe ZSM-5 et d'une zéolithe mordénite et comprenant du platine (comparatif).**

La zéolithe mordénite utilisée pour la préparation du catalyseur de cet exemple 1 est une zéolithe commerciale de la société Zeolyst. Elle présente un rapport Si/Al, mesuré par FX, égal à 10,0 et une teneur résiduelle en sodium égale à 109 ppm. Elle est sous sa forme acide.

La zéolithe ZSM-5 utilisée pour la préparation du catalyseur de cet exemple 1 est une zéolithe commerciale de la société Zeolyst. Elle présente un rapport Si/Al, mesuré par FX, égal à 17,5 et une teneur résiduelle en sodium égale à 132 ppm. Elle est sous sa forme acide.

La zéolithe ZSM-5 et la zéolithe mordénite, qui se trouvent à l'état de poudre, sont ensuite mélangées mécaniquement puis mises en forme par extrusion avec un gel d'alumine de manière à obtenir, après séchage à 100° C pendant une nuit et calcination à 500°C sous air sec, un support qui contient en poids 15 % de zéolithes (ZSM-5 + mordénite) et 85 % d'alumine. Les zéolithes ZSM-5 et mordénite sont présentes en une teneur pondérale équivalente dans le support (répartition pondérale de 50/50). Le support zéolithique est soumis à une imprégnation à sec par une solution d'acide hexachloroplatinique $H_2PtCl_6$ de manière à introduire 0,3 % poids de platine par rapport au poids du catalyseur. Le solide humide est ensuite séché à 120°C pendant 12 heures et calciné sous un débit d'air sec à la température de 500°C pendant une heure. Le catalyseur A ainsi obtenu contient en poids 7,5 % de zéolithe EU-1 sous forme H, 7,5 % de zéolithe ZSM-5 sous forme H, 84,7% d'alumine et 0,3% de platine.

**Exemple 2 : préparation de catalyseurs bizéolithiques dont une zéolithe est une zéolithe EU-1 et comprenant du platine et de l'indium (invention).**

Les zéolithes utilisées pour préparer les catalyseurs illustrant l'invention sont présentées dans le tableau 1 avec leur composition (rapport atomique Si / Al) et leur teneur résiduelle en sodium. Les zéolithes concernées sont sous forme acide.

Pour la zéolithe EU-1, on utilise comme matière première une zéolithe EU-1, brute de synthèse, comprenant le structurant organique, du silicium et de l'aluminium, possédant un rapport atomique Si/Al global égal à 13,6, une teneur pondérale en sodium par rapport au poids en zéolithe EU-1 sèche d'environ 1,5%, correspondant à un rapport atomique Na/Al de 0,6. Cette zéolithe EU-1 subit tout d'abord une calcination dite sèche à 550°C sous flux d'air durant 6 heures. Puis le solide obtenu est soumis à trois échanges ioniques dans une solution de $NH_4NO_3$ 10N, à environ 100°C pendant 4 heures pour chaque échange. A l'issue de ces traitements, la zéolithe EU-1 sous forme $NH_4$ a un rapport Si/Al atomique égal à 18,3, une teneur pondérale en sodium par rapport au poids de zéolithe EU-1 sèche de 50 ppm, correspondant à un rapport atomique Na/Al de 0,003.

Les zéolithes béta, mordénite et ZSM-5 sont des zéolithes commerciales (Zeolyst). La zéolithe IM-5 est synthétisée selon l'exemple n°1 de la demande de brevet EP-A-0.946.416 ou du brevet US 6.136.290 dont le contenu est incorporé ici par référence.

Tableau 1: caractéristiques des zéolithes utilisées pour l'invention

| zéolithes | Si/Al (FX) | Na (ppm) |
|-----------|-----------|----------|
| Béta | 12,5 | 87 |
| ZSM-5 | 17,5 | 132 |
| Mordénite | 10,0 | 109 |
| IM-5 | 12,0 | 84 |
| EU-1 | 18,3 | 50 |

Toutes les zéolithes se trouvent à l'état de poudre. La zéolithe EU-1, qui se trouvent à l'état de poudre, est ensuite mélangée mécaniquement avec un autre type de zéolithe (beta, ZSM-5, mordénite ou IM-5) puis l'ensemble est mis en forme par extrusion avec un gel d'alumine de manière à obtenir, après séchage à 100°C pendant une nuit et calcination à 500°C sous air sec, un support qui contient en poids 15 % de zéolithes (une zéolithe EU-1 en association avec un autre type de zéolithe) et 85 % d'alumine. La répartition pondérale des zéolithes dans le support zéolithique ainsi que le type de zéolithes présent dans chaque support sont donnés dans le tableau 2.

Pour la préparation des catalyseurs B, C, D et E, le support zéolithique comprenant un mélange de 2 zéolithes différentes (EU-1 + mordénite pour la catalyseur B, EU-1 + béta pour le catalyseur C, EU-1 + ZSM-5 pour le catalyseur D et EU-1 + IM-5 pour le catalyseur E) est d'abord soumis à une imprégnation à sec par une solution d'acide hexachloroplatinique $H_2PtCl_6$ de manière à introduire 0,3 % poids de platine par rapport au poids du catalyseur. Le solide ainsi imprégné est séché une nuit à 120°C puis est soumis à une imprégnation à sec par une solution de nitrure d'indium pour déposer 0,3% poids d'indium par rapport au poids du catalyseur. Le solide humide est ensuite séché à 120°C pendant 12 heures et calciné sous un débit d'air sec à la .température de 500°C pendant une heure. La composition des catalyseurs obtenus est reportée dans le tableau 2.

Le catalyseur F est préparé de la même manière que le catalyseur B, l'imprégnation par le nitrure d'indium étant immédiatement suivie d'une imprégnation à sec par une solution de chlorure d'étain de manière à introduire 0,3 % poids d'étain par rapport au poids du catalyseur F.

Tableau 2

| Catalyseur | zéolithe(s) | répartition des zéolithes | % poids métal |
|-----------|-------------|---------------------------|---------------|
| B | EU-1 + mordénite | 50/50 | 0,3% Pt, 0,3% In |
| C | EU-1 + béta | 75/25 | 0,3% Pt, 0,3% In |
| D | EU-1 + ZSM-5 | 75/25 | 0,3% Pt, 0,3% In |
| E | EU-1 + IM-5 | 75/25 | 0,3% Pt, 0,3% In |
| F | EU-1 + mordénite | 50/50 | 0,3% Pt, 0,3% In, 0,3% Sn |

**Exemple 3 : Evaluation des propriétés catalytiques des catalyseurs A à F en isomérisation d'une coupe C8 aromatique.**

Les performances des catalyseurs A à F ont été évaluées dans l'isomérisation d'une coupe aromatique comprenant des composés aromatiques à 8 atomes de carbone par molécule, principalement du métaxylène, de forthoxylène et de l'éthylbenzène. Les conditions opératoires utilisées sont:

-    Température = 390°C,
-    pression = 15 bar,
-    pression partielle $H_2$ = 12 bar

Les catalyseurs sont préalablement traités avec une charge contenant du dimethyldisulfure (DMDS) en présence d'hydrogène, avec une concentration telle que le rapport atomique soufre sur métal soit de 1,5. Les catalyseurs sont ensuite maintenus pendant 3 heures à 400°C sous débit d'hydrogène puis la charge est injectée.

Les vitesses spatiales horaires sont ajustées pour chaque catalyseur afin d'être à isoconversion de l'éthylbenzène. Les catalyseurs ont été comparés en terme de sélectivité par les pertes nettes à isoconversion de l'éthylbenzène.

La réaction d'isomérisation conduit à des réactions parasites générant trois types de pertes les pertes vers les paraffines résultant essentiellement des réactions d'ouverture de cycles naphténiques suivies de craquage, les pertes vers les aromatiques formés par les réactions de dismutation et de transalkylation des aromatiques à 8 atomes de carbone (AC8), et enfin les pertes vers les naphtènes dont les naphtènes à 8 atomes de carbone (N8) dus à l'hydrogénation des aromatiques. Les N8 pouvant être recyclés, on comparera les pertes par craquage et dismutation/transalkylation incluant les naphtènes autres que N8 (dont la somme constitue les pertes nettes).

Les pertes par craquage (P1) sont des pertes en AC8 sous forme de paraffines (PAR) de C1 à C8 :

$$P1(\%poids) = 100 \times [(\%PAR_{effluent} \times poids\ d'effluent) - (\%PAR_{charge} \times poids\ de\ charge)] / (\%AC8_{charge} \times poids\ de\ charge)$$

Les pertes par dismutation/transalkylation (P2) sont des pertes en AC8 sous forme de naphtènes autres que N8, de toluène, de benzène et de C9+ aromatiques (OAN) :

$$P2(\%poids) = 100 \times [(\%OAN_{effluent} \times poids\ d'effluent) - (\%OAN_{charge} \times poids\ de\ charge)] / (\%AC8_{charge} \times poids\ de\ charge)$$

La somme des pertes P1 et P2 représente les pertes nettes.

Les données présentées dans le tableau 3 ont été obtenues à iso-conversion de l'éthybenzène (61 %).

Tableau 3: évaluation catalytique des catalyseurs A à F

| Catalyseur | A | B | C | D | E | F |
|---|---|---|---|---|---|---|
| pertes nettes (% poids) | 4,9 | 4,1 | 4,2 | 4,4 | 4,5 | 4,1 |

Les catalyseurs B à F selon l'invention qui comprennent chacun une zéolithe de type structural EUO et une zéolithe choisie parmi les zéolithes mordénite (catalyseurs B et F), beta (catalyseur C), ZSM-5 (catalyseur D) et IM-5 (catalyseur E) ainsi que du platine et de l'indium, conduisent à une diminution des pertes nettes traduisant des réactions secondaires limitées par rapport aux performances obtenues au moyen du catalyseur A dépourvu d'indium.

**Revendications**

1. Catalyseur comportant au moins une zéolithe de type structural EUO, au moins une zéolithe choisie parmi la zéolithe IM-5 et les zéolithes de type structural MFI, MOR, BEA et MTW, au moins un métal du groupe VIII, au moins un métal du groupe IIIA et au moins une matrice minérale poreuse.

2. Catalyseur selon la revendication 1 dans lequel la zéolithe de type structural EUO est une zéolithe EU-1.

3. Catalyseur selon la revendication 1 ou la revendication 2 dans lequel la zéolithe choisie parmi la zéolithe IM-5 et les zéolithes de type structural MFI, MOR, BEA et MTW est choisie parmi les zéolithes IM-5, ZSM-5, mordénite, beta et ZSM-12.

4. Catalyseur selon l'une des revendications 1 à 3 dans lequel le métal du groupe VIII est le platine.

5. Catalyseur selon l'une des revendications 1 à 4 dans lequel le métal du groupe IIIA est l'indium.

6. Catalyseur selon l'une des revendications 1 à 5 comportant au moins un métal du groupe IVA.

7. Catalyseur selon la revendication 6 dans lequel ledit métal du groupe IVA est l'étain.

8. Catalyseur selon l'une des revendications 1 à 7 comportant du soufre.

9.  Procédé d'isomérisation d'une charge comprenant des composés aromatiques à 8 atomes de carbone par molécule réalisé en présence d'un catalyseur selon l'une des revendications 1 à 8.

10. Procédé d'isomérisation selon la revendication 9 tel que ladite charge comprend un mélange de xylènes et de l'éthylbenzène.

11. Procédé d'isomérisation selon la revendication 9 ou la revendication 10 tel qu'il est réalisé à une température comprise entre 300 et 500°C, avec une pression partielle d'hydrogène comprise entre 0,3 et 1,5 MPa, avec une pression totale comprise entre 0,45 et 1,9 MPa et une vitesse spatiale d'alimentation, exprimée en kilogramme de charge introduite par kilogramme de catalyseur et par heure, comprise entre 0,25 et 30 h$^{-1}$.


**Claims**

1.  A catalyst comprising at least one zeolite with structure type EUO, at least one zeolite selected from IM-5 zeolite and zeolites with structure type MFI, MOR, BEA and MTW, at least one group VIII metal, at least one group IIIA metal and at least one porous mineral matrix.

2.  A catalyst according to claim 1, in which the zeolite with structure type EUO is an EU-1 zeolite.

3.  A catalyst according to claim 1 or claim 2, in which the zeolite selected from IM-5 zeolite and zeolites with structure type MFI, MOR, BEA and MTW is selected from IM-5, ZSM-5, mordenite, beta and ZSM-12 zeolites.

4.  A catalyst according to one of claims 1 to 3, in which the group VIII metal is platinum.

5.  A catalyst according to one of claims 1 to 4, in which the group IIIA metal is indium.

6.  A catalyst according to one of claims 1 to 5, comprising at least one group IVA metal.

7.  A catalyst according to claim 6, in which said group IVA metal is tin.

8.  A catalyst according to one of claims 1 to 7, comprising sulphur.

9.  A process for isomerizing a feed comprising aromatic compounds containing eight carbon atoms per molecule, carried out in the presence of a catalyst in accordance with one of claims 1 to 8.

10. An isomerization process according to claim 9, in which said feed comprises a mixture of xylenes and ethylbenzene.

11. An isomerization process according to claim 9 or claim 10, carried out at a temperature in the range 300°C to 500°C with a partial pressure of hydrogen in the range 0.3 to 1.5 MPa, with a total pressure in the range 0.45 to 1.9 MPa and at an hourly space velocity, expressed in kilograms of feed introduced per kilogram of catalyst per hour, in the range 0.25 to 30 h$^{-1}$.


**Patentansprüche**

1.  Katalysator, der mindestens einen Zeolithen des Strukturtyps EUO, mindestens einen Zeolithen, ausgewählt aus IM-5-Zeolith und den Zeolithen des Strukturtyps MFI, MOR, BEA und MTW, und mindestens ein Metall der Gruppe VIII, mindestens ein Metall der Gruppe IIIA und mindestens eine poröse mineralische Matrix umfasst.

2.  Katalysator nach Anspruch 1, wobei der Zeolith des Strukturtyps EUO ein EU-1-Zeolith ist.

3.  Katalysator nach Anspruch 1 oder Anspruch 2, wobei der Zeolith, der aus IM-5-Zeolith und den Zeolithen des Strukturtyps MFI, MOR, BEA und MTW ausgewählt ist, aus den IM-5-, ZSM-5-, Mordenit-, Beta- und ZSM-12-Zeolithen ausgewählt ist.

4.  Katalysator nach einem der Ansprüche 1 bis 3, wobei das Metall der Gruppe VIII Platin ist.

5. Katalysator nach einem der Ansprüche 1 bis 4, wobei das Metall der Gruppe IIIA Indium ist.

6. Katalysator nach einem der Ansprüche 1 bis 5, der mindestens ein Metall der Gruppe IVA umfasst.

7. Katalysator nach Anspruch 6, wobei das Metall der Gruppe IVA Zinn ist.

8. Katalysator nach einem der Ansprüche 1 bis 7, der Schwefel umfasst.

9. Verfahren zur Isomerisierung einer Beschickung, die aromatische Verbindungen mit 8 Kohlenstoffatomen je Molekül umfasst, das in Gegenwart eines Katalysators nach einem der Ansprüche 1 bis 8 ausgeführt wird.

10. Verfahren zur Isomerisierung nach Anspruch 9, so dass die Beschickung eine Mischung aus Xylenen und Ethylbenzen umfasst.

11. Verfahren zur Isomerisierung nach Anspruch 9 oder Anspruch 10, so dass es bei einer Temperatur im Bereich zwischen 300 und 500 °C, mit einem partiellen Wasserstoffdruck im Bereich zwischen 0,3 und 1,5 MPa, mit einem Gesamtdruck im Bereich zwischen 0,45 und 1,9 MPa und einer Versorgungsraumgeschwindigkeit, ausgedrückt in Kilogramm eingeführter Beschickung pro Kilogramm Katalysator und pro Stunde, im Bereich zwischen 0,25 und 30 $h^{-1}$ ausgeführt wird.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0923987 A1
- EP 42226 B1
- US 4640829 A
- EP 51318 A1
- EP 0946416 B
- US 6136290 A
- EP 0946416 A

**Littérature non-brevet citée dans la description**

- **W.M. MEIER ; D.H. OLSON.** Atlas of Zeolite Framework Types. 2001
- **N.A. BRISCOE et al.** *Zeolites,* 1988, vol. 8, 74
- **W. M MEIER ; D. H. OLSON ; CH. BAERLOCHER.** Atlas of Zeolite Framework Types. 2001